# EUROPÄISCHE PATENTSCHRIFT

(11) **EP 4 129 189 B1**
(45) Veröffentlichungstag und Bekanntmachung des Hinweises auf die Patenterteilung: **22.04.2026**
(21) Anmeldenummer: 22186128.9
(22) Anmeldetag: 20.07.2022
(51) Int. Cl.: A61B 6/03, A61B 6/12, A61B 6/00

(54) **VERFAHREN ZUM BETREIBEN EINES MEDIZINISCHEN BILDGEBUNGSGERÄTS ZUR LAGERICHTIGEN DARSTELLUNG VON NICHT-ANATOMISCHEN STRUKTUREN WÄHREND EINER BILDGEBENDEN UNTERSUCHUNG UND VORRICHTUNG HIERFÜR**
METHOD FOR OPERATING A MEDICAL IMAGING DEVICE FOR THE CORRECT PRESENTATION OF NON-ANATOMICAL STRUCTURES DURING AN IMAGING EXAMINATION AND DEVICE THEREFOR
PROCÉDÉ PERMETTANT DE FAIRE FONCTIONNER UN APPAREIL D'IMAGERIE MÉDICALE DESTINÉ À LA REPRÉSENTATION DANS UNE POSITION CORRECTE DES STRUCTURES NON ANATOMIQUES LORS D'UN EXAMEN D'IMAGERIE ET DISPOSITIF CORRESPONDANT

(30) Priorität: 02.08.2021 DE 102021003956
(43) Veröffentlichungstag der Anmeldung: 08.02.2023
(73) Patentinhaber: Ziehm Imaging GmbH, 90471 Nürnberg (DE)
(72) Erfinder: Vöth, Tim, 90459 Nürnberg (DE); König, Thomas, 90478 Nürnberg (DE); Hörndler, Klaus, 90482 Nürnberg (DE); Knaupp, Michael, 91244 Reichenschwand (DE); KACHELRIEß, Marc, 90419 Nürnberg (DE)
(74) Vertreter: Wittmann, Günther

(56) Entgegenhaltungen:
- US-A1- 2020 410 666
- US-A1- 2021 128 011

## Beschreibung

Die Erfindung betrifft ein Verfahren zum Betreiben eines medizinischen Bildgebungsgeräts zur lagerichtigen Darstellung von nicht-anatomischen Strukturen während einer bildgebenden Untersuchung. Die Erfindung umfasst weiterhin ein medizinisches Bildgebungsgerät.

Bei medizinischen chirurgischen Eingriffen, beispielsweise in der vaskulären Chirurgie, wird angestrebt, diese Eingriffe in das Körperinnere eines zu behandelnden Patienten minimalinvasiv durchzuführen, da eine solche minimalinvasive Vorgehensweise eine möglichst geringe Belastung für den zu behandelnden Patienten darstellt. Der Zugang zum Körperinneren erfolgt dabei über kleine, durch den behandelnden Arzt vorgenommene Einschnitte oder alternativ über Körperöffnungen des Patienten. Weil das Zielgebiet eines solchen Eingriffs für den behandelnden Arzt schwer bzw. in einer Vielzahl von Eingriffen nicht einsehbar ist, werden nicht-anatomische Strukturen, insbesondere Interventionsmaterialien, beispielsweise Führungsdrähte, Katheter, Stents, Coils, Schrauben, d.h. Strukturen welche für den Eingriff in den Patienten eingeführt werden, und die umgebende Patientenanatomie (anatomische Strukturen) durch bildgebende Verfahren visualisiert. Oftmals werden hierfür projektive medizinische Bildgebungsverfahren angewendet, vorzugsweise 2D-Verfahren, d.h. Verfahren, die die dreidimensionale Struktur eines abzubildenden Volumens auf eine zweidimensionale Fläche projizieren und somit 2D-Bilder erzeugen. Vorzugsweise kommt hier die 2D-Röntgenfluoroskopie zum Einsatz, d.h. es werden 2D-Bilder, insbesondere 2D-Röntgenbilder, in einer zeitlichen Abfolge aufgenommen und auf einem Anzeigegerät angezeigt. Die Information über die räumliche (3D-) Beschaffenheit des Patienten und beispielsweise des Interventionsmaterials, d.h. deren dreidimensionale Gestalt, geht dabei größtenteils verloren, da alle Strukturen, die auf einem Projektionsstrahl liegen, beispielsweise einem Röntgenstrahl, auf einen Punkt einer Fläche projiziert werden, d.h. diese Strukturen erscheinen auf einem 2D-Bild überlagert. 2D-Bilder bestehen aus Pixeln, welchen unterschiedliche Werte, beispielsweise ganzzahlige Werte, beispielsweise 1 oder 0, zugeordnet werden können. Somit wird eine Struktur, welche auf einem Projektionsstrahl liegt, beispielsweise einem Röntgenstrahl, auf einen Pixel eines 2D-Bildes projiziert, wobei die Werte von Pixeln von der Art und Beschaffenheit der zu projizierenden Strukturen abhängig sind. Würde dem behandelnden Arzt eine zeitlich schnelle Abfolge von 3D-Rekonstruktionen vorliegen, welche die eingebrachten, nicht-anatomischen-Strukturen, beispielsweise Interventionsmaterialien, und die Patientenanatomie in einer 3D-Ansicht zeigt, erhielte dieser eine 4D-Ansicht, d. h. drei räumliche plus eine zeitliche Dimension. Dies bedeutet, dem behandelnden Arzt würde für die Durchführung des Eingriffs eine 4D-Interventionsführung zur Verfügung stehen, welche es ermöglicht, die Lage des in den Patienten eingebrachten Interventionsmaterials zu einem aktuellen Zeitpunkt zu beurteilen.

Für die Zurverfügungstellung einer 3D-Rekonstruktion ist die Aufnahme bzw. die Anfertigung einer Tomographie nötig, welche beispielsweise mittels eines Computertomographen (CT), eines C-Bogen-Röntgengerätes (wobei das C-Bogen-Röntgengerät ein mobiles oder stationär installiertes C-Bogen-Röntgengerät sein kann), oder mittels eines Magnetresonanztomographen durchgeführt wird. Eine 3D-Rekonstruktion ist ein 3D-Volumen, welches aus einer Vielzahl von Einzelmessungen berechnet wird. In der Computertomographie handelt es sich bei den Einzelmessungen um 2D-Röntgenprojektionen. Die für eine entsprechende kontinuierliche Erfassung des Patienten und des Interventionsmaterials notwendige kontinuierliche aufeinanderfolgende Durchführung vieler 3D-Röntgenaufnahmen geht mit einer sehr hohen Strahlenbelastung für den Patienten einher. Dies macht einen gegenwärtigen Einsatz von 4D-Interventionsführung in der Chirurgie, insbesondere in der vaskulären Chirurgie, unmöglich. Bei einem Computertomographen kann es sich um jedes Bildgebungsgerät handeln, welches 2D-Projektionsbilder aufnimmt und daraus eine Tomographie berechnet.

Der Stand der Technik zur Führung von Interventionsmaterialen, insbesondere in Blutgefäßen, umfasst eine Vielzahl von Verfahren, dabei liegt insbesondere röntgenbasierten Bildgebungsverfahren die Problematik zu Grunde, dass Blutgefäße im Vergleich zum umliegenden Gewebe einen nahezu gleichen Kontrast aufweisen und somit, insbesondere auf Röntgenbildern oder Computertomographien, zunächst nicht sichtbar sind. Bei der Aufnahme von Röntgenbildern bzw. Computertomographien ist daher die Gabe eines Kontrastmittels in das Gefäßsystem notwendig, beispielsweise Jod oder Kohlenstoffdioxid, welches eine höhere bzw. niedrigere Röntgenabsorption als das umliegende Gewebe zeigt und somit das Gefäßsystem sichtbar macht. Bei der Gabe von Kontrastmittel in den Patienten kann ein weiterer Effekt sein, dass auch minder- bzw. nichtdurchblutete Gefäße des Gefäßsystems dem behandelnden Chirurgen auffallen können.

Kontrastmittelhaltige Aufnahmen können in einer Vielzahl von Verfahren verwendet werden. Bei subtraktionsbasierten 2D-Bildgebungsverfahren können sie nach Abzug eines Bildes ohne Kontrastmittel (Maske) die Dynamik des Blutflusses oder die Form eines Gefäßsystems darstellen, beispielsweise in der Digitalen Subtraktionsangiografie (DSA). Auch können sie dafür verwendet werden, eine Spur einzublenden, um ein Instrument in einem Gefäß bzw. einem Gefäßsystem zu führen. Jedoch muss hier bei jeder Änderung der Aufnahmegeometrie oder Bewegung des Patienten eine erneute Kontrastmittelgabe erfolgen, einhergehend mit einer erhöhten Strahlenbelastung. Die wiederholte Zugabe von jodhaltigem Kontrastmittel kann unter Umständen auch toxisch auf die Nieren wirken und damit bei einer Niereninsuffizienz kontraindiziert sein. Darüber hinaus geht hier die Tiefeninformation in Form der dritten Dimension in der in dem vorangegangenen Abschnitt beschriebenen Form verloren.

Es empfiehlt sich daher die Verwendung einer 3D-Rekonstruktion der Gefäße, welche beispielsweise aus einer zuvor aufgenommenen kontrastmittelgefüllten Computertomographie extrahiert werden kann und beispielsweise als Kontur jeweils lagerichtig unter Bestimmung und Verwendung der korrekten Projektionsgeometrie in ein Live-2D-Bild eingeblendet wird.

Die im vorangegangen Abschnitt beschriebenen Verfahren offenbaren den Nachteil, dass die kontrastmittelgefüllten Gefäße und, sofern vorhanden, die nicht-anatomischen Strukturen nur auf einem Live-2D-Bild für den behandelnden Arzt erkennbar sind. Auch bei der Verwendung mehrerer Röntgengeräte, insbesondere C-Bogen-Röntgengeräte, insbesondere mobile-C-Bogen-Röntgengeräte, welche unterschiedliche Projektionsrichtungen, beispielsweise orthogonale Projektionsrichtungen aufweisen, steht keine vollständige 3D-Information zur Verfügung. Es kann daher eine erhebliche Menge an Zeit, Strahlendosis und Kontrastmittel benötigt werden, um beispielsweise einen Führungsdraht korrekt innerhalb eines Gefäßsystems zu navigieren, was eine enorme Belastung für den zu behandelnden Patienten darstellt.

Das Dokument EP2656314B1 offenbart ein Verfahren und ein System, welche basierend auf einer komprimierten Abtastung eine radiologische Führung eines Instruments während einer medizinischen Untersuchung ermöglichen.

Die DE 10 2008 054 298 A1 offenbart ein Verfahren und eine Vorrichtung zur 3-D-Visualisierung eines Eingriffspfades eines medizinischen Instrumentes, eines medizinischen Instrumentes und/oder einer bestimmten Gewebestruktur eines Patienten zur Unterstützung eines medizinischen Eingriffs an einem Gewebe eines Patienten. Bei dem Verfahren und mit der Vorrichtung wird basierend auf zwei unter voneinander verschiedenen Projektionsrichtungen mit einer Röntgeneinrichtung erzeugten 2-D-Röntgenbildern von dem das Gewebe des Patienten und/oder das medizinische Instrument und/oder die bestimmte Gewebestruktur aufweisenden Körperbereich ein Eingriffspfad festgelegt und/oder das medizinische Instrument und/oder die bestimmte Gewebestruktur identifiziert. Der Eingriffspfad und/oder das medizinische Instrument und/oder die bestimmte Gewebestruktur werden in ein 3-D-Patientenrechenmodell übernommen, welches das Gewebe als Modellgewebe aufweist, und es wird zumindest ein Ausschnitt des 3-D-Patientenrechenmodells oder des Modellgewebes mit eingeblendetem Modelleingriffspfad und/oder Modellinstrument und/oder Modellgewebestruktur auf einem Sichtgerät angezeigt.

Der Stand der Technik weist den Nachteil auf, dass die für die 3D-Rekonstruktionen der Interventionsmaterialien zu einem bestimmten Zeitpunkt benötigten 2D-Röntgenprojektionen gleichzeitig oder nahezu gleichzeitig (pseudogleichzeitig) aufgenommen werden müssen, um die Interventionsmaterialien bei Vorliegen von Bewegungen, insbesondere verursacht durch Führung eines Instruments durch den behandelnden Arzt, korrekt wiederzugeben. Liegt hingegen zwischen den Aufnahmen einzelner oder mehrerer 2D-Röntgenprojektionen ein zeitlicher Versatz, so ist im Allgemeinen keine konsistente 3D-Rekonstruktion zu einem bestimmten Zeitpunkt möglich. Je nach Stärke der auftretenden Bewegung können hierbei Bewegungsartefakte auftreten, oder die 3D-Rekonstruktionen komplett untauglich werden. Ein solcher zeitlicher Versatz entsteht insbesondere dann, wenn das zur Aufnahme verwendete Röntgengerät zu wenige Bildketten, also Paare aus Röntgenquelle und - detektor aufweist, da hierbei das Röntgensystem zwischen der Aufnahme von 2D-Aktualisierungsprojektionen die Aufnahmegeometrie verändern muss, beispielsweise durch Rotation einer Gantry. Erfolgt diese Rotation dabei nicht schnell genug, ergibt sich damit der beschriebene Nachteil der inkonsistenten und ggf. untauglichen 3D-Rekonstruktion.

Die US 2021/0128011 A1 offenbart ein bildgebendes Verfahren zur radiologischen Führung eines Instruments bei medizinischen Eingriffen an einem Objekt, umfassend: a) Bereitstellen eines ersten Bild des Objekts gefolgt von b) der Bereitstellung aktualisierter Bilder während des während des Eingriffs an einen Bediener durch Messung eines unterabgetasteten Satz von Projektionen des Objekts und Rekonstruktion des aktualisierten Bildes auf der Grundlage von Änderungen zwischen dem ersten Bild oder einer Aktualisierung des ersten Bildes und dem unterabgetasteten Satz von Projektionen. Dokument D1 betrifft ferner besondere Einsätze des Verfahrens und ein System zum radiologischen Leiten medizinischer Eingriffe an einem Objekt gemäß dem Verfahren, umfassend Mittel zum Bereitstellen eines ersten Bildes des Objekts, eine Abbildungsvorrichtung, die unterabgetastete Sätze von Projektionen misst, Verarbeitungsmittel, die mit der Abbildungsvorrichtung in Verbindung stehen, um während des Eingriffs aktualisierte Bilder zu liefern, indem das aktualisierte Bild auf der Grundlage von Änderungen zwischen dem ersten Bild oder einer Aktualisierung des ersten Bildes und dem unterabgetasteten Satz von Projektionen rekonstruiert wird.

Eine dem Fachmann naheliegende Lösung dieses Problems besteht nun darin, eine sehr schnelle Änderung der Aufnahmegeometrie beispielsweise mittels einer schnell rotierenden Gantry zu ermöglichen. Dies stellt jedoch hohe Anforderungen sowohl an die mechanische Lagerung des Röntgensystems, beispielsweise einer Gantry, und der darin verbauten Teile, als auch an die zur Aufnahme von 2D-Röntgenprojektionen benötigten Komponenten. Um nämlich die Bewegungsunschärfe in den aufgenommenen 2D-Röntgenprojektionen, welche durch die schnelle Änderung der Aufnahmegeometrie während der Belichtung der Röntgendetektoren entsteht, gering zu halten, muss die Röntgenquelle in sehr kurzen, aber vergleichsweise intensiven Pulsen strahlen, und/oder muss die Aufnahmerate der Röntgendetektoren sehr hoch sein. Dies erhöht im Allgemeinen die Kosten eines solchen Systems bzw. ist im Falle der Verwendung von Röntgen-Flachdetektoren technisch nicht möglich.

Es ist somit Aufgabe der vorliegenden Erfindung, ein verbessertes Verfahren zur lagerichtigen Darstellung von nicht-anatomischen-Strukturen und anatomischen Strukturen während einer bildgebenden Untersuchung bereitzustellen.

Die Aufgabe der Erfindung wird erfindungsgemäß durch die Gegenstände der unabhängigen Ansprüche gelöst. Vorteilhafte Ausbildungen sind in den abhängigen Patentansprüchen angegeben.

Dem erfindungsgemäßen Verfahren liegt die Zurverfügungstellung eines ersten 3D-Bildes (3D-Volumen) zugrunde. Dieses erste 3D-Bild wird entweder während eines interventionellen Eingriffs mittels einer 3D-Aufnahme aufgenommen oder beispielsweise aus einem Patientenarchiv geladen, wobei im Fall des Ladens des ersten 3D-Bildes aus einem Patientenarchiv der erste 3D-Scan präoperativ aufgenommen wurde. Das erste 3D-Bild, welches wenigstens eine anatomische Struktur, enthält, kann beispielsweise durch eine präoperative Computertomographie- oder Magnetresonanztomographie-Aufnahme angefertigt werden. Die wenigstens eine anatomische Struktur des ersten 3D-Bildes kann beispielsweise teilweise Knochen- und/oder teilweise Gefäßstrukturen sowie umgebende Anatomie, beispielsweise Organe, Muskeln oder andere Weichteile beinhalten. Ferner kann das erste 3D-Bild weitere nicht-anatomische Strukturen, beispielsweise Implantate oder Interventionsmaterialien aus vorangegangenen chirurgischen Eingriffen und weitere anatomische Strukturen aufweisen, beispielsweise Hautkanten. Alternativ kann das erste 3D-Bild auch intraoperativ aufgenommen werden, vorzugsweise unter Verwendung eines intraoperativen Computertomographen oder eines C-Bogen-Röntgengeräts, insbesondere mit einem mobilen C-Bogen-Röntgengerät. Vorzugsweise wird das erste 3D-Bild mittels dem Gerät aufgenommen, welches im Anschluss für eine 4D-Interventionsführung verwendet wird. Ferner besteht die Möglichkeit, das erste 3D-Bild in eine interne Speichereinheit, beispielsweise einen internen Bilddatenspeicher, oder eine externe Speichereinheit, beispielsweise einen USB-Stick, eine externe Festplatte, oder einen Onlinespeicher, auf welche das das erfindungsgemäße Verfahren ausführende Bildgebungsgerät, vorzugsweise ein Röntgengerät, Zugriff hat, zu importieren. Bereits vor dem Import oder im Anschluss an diesen wird ein Anatomiemodell der wenigstens einen anatomischen Struktur des ersten 3D-Bildes extrahiert. Erfindungsgemäß ist eine Extraktion eine Berechnung eines Anatomiemodells, wobei das Modell beispielsweise einer voxelbasierten Segmentierung oder einer parametrischen Repräsentation entsprechen kann. Handelt es sich beispielsweise bei der wenigstens einen anatomischen Struktur insbesondere um ein Organ, vorzugsweise um ein Hohlorgan, beispielsweise ein kontrastmittelhaltiges Blutgefäß oder eine kontrastmittelhaltige Herzkammer oder einen Darm, so kann beispielsweise ein Modell der Oberfläche des Hohlorgans berechnet werden. Handelt es sich bei der wenigstens einen anatomischen Struktur beispielsweise um einen Knochen, beispielsweise einen Wirbelkörper, so kann beispielsweise eine Segmentierung aller zugehörigen Voxel der anatomischen Struktur berechnet werden.

Erfindungsgemäß ist es vorgesehen, dass anschließend wenigstens zwei 2D-Aktualisierungsbilder zur Verfügung gestellt werden, wobei die 2D-Aktualisierungsbilder wenigstens teilweise anatomische Strukturen und/oder wenigstens teilweise nicht-anatomische Strukturen eines Untersuchungsbereiches enthalten. Die wenigstens teilweise anatomischen Strukturen können dabei Teile der Knochenstrukturen oder Teile der Gefäßstrukturen oder Teile von Knochen- und Gefäßstrukturen sein. Die nicht-anatomischen Strukturen sind insbesondere in den zu behandelnden Patienten eingebrachte Interventionsmaterialien, beispielsweise Führungsdrähte, Katheter, Stents, Coils oder Schrauben. Bei den zur Verfügung gestellten wenigstens zwei 2D-Aktualisierungsbildern kann es sich teilweise um neu aufgenommene 2D-Aktualisierungsbilder und teilweise um 2D-Aktualisierungsbilder, die während des chirurgischen Eingriffs aufgenommen wurden und zur Berechnung einer 3D-Rekonstruktion benutzt wurden, handeln. Die wenigstens zwei 2D-Aktualisierungsbilder, können mittels eines Röntgengerätes, beispielsweise eines C-Bogen-Röntgengerätes oder Computertomographen (gantrybasiertes System) aufgenommen worden sein, wobei die nicht-anatomischen Strukturen und/oder die anatomischen Strukturen eine Veränderung ihrer Lage während des chirurgischen Eingriffs erfahren haben können, wobei die nicht-anatomischen Strukturen beispielsweise durch eine Bewegung innerhalb des Patienten, beispielsweise durch das Weiterführen eines Führungsdrahtes, bewegt werden können. Die anatomischen Strukturen können beispielsweise eine Veränderung ihrer Lage aufgrund der Atmung des Patienten oder durch ein Verrücken des Patienten durch den behandelnden Chirurgen erfahren haben. Bei den zur Verfügung gestellten, insbesondere den neu zur Verfügung gestellten, d.h. zuletzt aufgenommenen 2D-Aktualisierungsbildern, handelt es sich vorzugsweise um in einem Zeitintervall aufgenommene 2D-Aktualisierungsbilder, insbesondere um nahezu gleichzeitig ("pseudogleichzeitig") aufgenommene wenigstens zwei 2D-Aktualisierungsbilder, vorzugsweise um gleichzeitig, d. h. zu einem Zeitpunkt, aufgenommene 2D-Aktualisierungsbilder. In Varianten des Verfahrens, in denen 2D-Aktualisierungsbilder nicht gleichzeitig aufgenommen werden, können die 2D-Aktualisierungsbilder beispielsweise mit einer einzigen Aufnahmevorrichtung, d.h. einer einzigen Bildkette, aufgenommen werden. Erfindungsgemäß werden wenigstens zwei der zur Verfügung gestellten 2D-Aktualisierungsbilder zu unterschiedlichen Zeitpunkten mit unterschiedlichen Blickrichtungen aufgenommen, da für die Berechnung einer 3D-Rekonstruktion der nicht-anatomischen Strukturen in der Regel mehr 2D-Aktualisierungsbilder benötigt werden als Bildketten vorhanden sind.

Im Anschluss an die Zurverfügungstellung der wenigstens zwei 2D-Aktualisierungsbilder erfolgt eine Extraktion von nicht-anatomischen Strukturen aus einer ersten Teilmenge der wenigstens zwei 2D-Aktualisierungsbilder, wobei die erste Teilmenge der wenigstens zwei 2D-Aktualisierungsbilder beispielsweise aus Null, einem, zwei, drei etc. 2D-Aktualisierungsbildern bestehen kann. Die erfindungsgemäße erste Teilmenge ist insbesondere unterschiedlich zu potentiellen weiteren erfindungsgemäßen Teilmengen, indem sie sich komplett oder teilweise, d.h. in wenigstens einem 2D-Aktualisierungsbild, voneinander bezüglich der beinhalteten 2D-Aktualisierungsbilder unterscheiden, wobei die Anzahl der 2D-Aktualisierungsbilder in beiden Teilmengen unterschiedlich sein kann. Die Extraktion kann dabei beispielsweise einer diskreten, pixelweisen Segmentierung entsprechen. In einer vorteilhaften Ausgestaltung der Erfindung kann die Extraktion dabei kontinuierliche Werte darstellen, insbesondere Beiträge der nicht-anatomischen Strukturen zu den 2D-Aktualisierungsbildern und hier insbesondere physikalisch korrekte oder näherungsweise korrekte Linienintegrale entlang der Projektionsstrahlen.

Erfindungsgemäß erfolgt ferner eine Extraktion von anatomischen Strukturen aus einer zweiten Teilmenge der 2D-Aktualisierungsbilder. Diese Extraktion anatomischer Strukturen kann dabei auch auf eine Weise erfolgen, in welcher die extrahierten nicht-anatomischen Strukturen aus den 2D-Aktualisierungsbildern gemäß ihres Beitrags zu diesen entfernt werden, z.B. durch Subtraktion. In einer vorteilhaften Ausgestaltung der Erfindung ist es vorgesehen, die Extraktion der anatomischen Strukturen so durchzuführen, dass an den Stellen nicht-anatomischer Strukturen keine sicht- oder messbaren Lücken in den resultierenden Bildern auftreten, entsprechend beispielsweise Luft, sondern dass die Extraktion auf eine Weise vollzogen wird, bei welcher deren Ergebnis demjenigen Fall entspricht, der vorläge, wäre überhaupt keine nicht-anatomische Struktur an den entsprechenden Stellen vorhanden und die entsprechenden Pixel durch das Gewebe besetzt, das die entsprechenden Stellen umgibt.

Im Anschluss an die Extraktion der nicht-anatomischen Strukturen aus der ersten Teilmenge der 2D-Aktualisierungsbilder erfolgt eine Berechnung eines nicht-anatomischen 3D-Bildes aus wenigstens zwei Teil-Rekonstruktionen, wobei die wenigstens zwei Teil-Rekonstruktionen aus den Extraktionen berechnet werden. Die Menge der 2D-Aktualisierungsbilder, die zur Verfügung gestellt werden kann, enthält aufgrund der begrenzten Anzahl an möglichen Aufnahmevorrichtungen in der Regel eine nicht ausreichende Anzahl von 2D-Aktualisierungsbildern, die gleichzeitig bzw. pseudogleichzeitig aufgenommen wurden. Um somit aus der Menge der gleichzeitig bzw. pseudogleichzeitig aufgenommenen 2D-Aktualisierungsbilder nach erfolgter Extraktion ein nicht-anatomisches 3D-Bild zu rekonstruieren, enthält die erste Teilmenge vorzugsweise 2D-Aktualisierungsbilder, welche zu unterschiedlichen Zeitpunkten aufgenommen wurden. Folglich stellen auch die Extraktionen verschiedene Zeitpunkte dar und können somit aufgrund möglicher Bewegung des Patienten und/oder der nicht-anatomischen Strukturen verschiedene Zustände, der nicht-anatomischen Strukturen darstellen, insbesondere verschiedene Positionen und Orientierungen. Um zu berücksichtigen, dass die Extraktionen unterschiedliche Zustände der nicht-anatomischen Strukturen darstellen können, werden zunächst nur solche Extraktionen in ein gemeinsames 3D-Volumen, erfindungsgemäß als Teil-Rekonstruktion bezeichnet, zurückgerechnet (vorzugsweise unter Verwendung einer Projektionsgeometrie, unter welcher die 2D-Aktualisierungsbilder aufgenommen wurden), die gleichzeitig oder pseudo-gleichzeitig aufgenommen wurden. Diese gleichzeitig bzw. pseudo-gleichzeitig aufgenommenen Bilder stammen dabei insbesondere aus unterschiedlichen im System vorhandenen Aufnahmevorrichtungen, insbesondere Bildketten, beispielsweise Paaren von Röntgenquelle und -detektor. Somit ist jede Teil-Rekonstruktion in sich und bezüglich des dargestellten Zustands der nicht-anatomischen Strukturen konsistent. Allerdings können im Allgemeinen weder einzelne Teil-Rekonstruktionen noch deren Kombination als taugliches nicht-anatomisches 3D-Bild verstanden werden, denn die Teil-Rekonstruktionen enthalten Artefakte, welche dadurch bedingt sind, dass die Anzahl der jeder Teil-Rekonstruktion zugrundeliegenden Extraktionen gering ist, beispielsweise kann für die Berechnung der Teil-Rekonstruktionen eine gefilterte oder ungefilterte Rückprojektion verwendet werden. Die Zusammenführung mehrerer Teil-Rekonstruktionen nach herkömmlicher, in der Computertomographie bekannten Verfahren erlaubt somit noch keine korrekte Darstellung der nicht-anatomischen Strukturen. Erfindungsgemäß ist es jedoch möglich, die Teil-Rekonstruktionen mittels solcher Bildverarbeitungsoperationen zusammenzuführen, welche das Entfernen dieser Artefakte zum Zweck haben. Um die Rechenzeit für ein solches Verfahren möglichst gering zu halten können dabei vorzugsweise Rechenoperationen verwendet werden, die ausnutzen, dass sowohl die Teil-Rekonstruktionen als auch das nicht-anatomische 3D-Bild in der Regel größtenteils leer sind, da in der Regel nur ein geringer Teil des Untersuchungsbereichs nicht-anatomische Strukturen enthält. Für das Entfernen der Artefakte der Teil-Rekonstruktionen können vorzugsweise maschinelle Lernverfahren zum Einsatz kommen, die mittels einer Vielzahl unkorrigierter Eingangsbilder, insbesondere einer Anzahl an Teil-Rekonstruktionen, lernen, korrigierte Ausgangbilder, insbesondere korrekte nicht-anatomische 3D-Bilder, zu rekonstruieren, welche nun keine Artefakte aufgrund einer zu geringen Anzahl an 2D-Aktualisierungsbilder enthalten. Maschinelle Lernverfahren können hier insbesondere dadurch erfolgreich sein, dass die nicht-anatomischen Strukturen in der Regel eine hohe Symmetrie, beispielsweise zylindrisch im Falle eines Führungsdrahts, aufweisen, was ein Erlernen ihrer tatsächlichen Form möglich macht.

Es kann seriell oder parallel zur Berechnung des nicht-anatomischen-3D-Bildes eine Rekonstruktion eines anatomischen-3D-Bildes aus den anatomischen Strukturen, welche aus der zweiten Teilmenge der 2D-Aktualisierungsbilder extrahiert wurden, erfolgen. Das anatomische 3D-Bild zeigt vorzugsweise nur die Patientenanatomie, nicht aber die nicht-anatomischen Strukturen. Dieses anatomische 3D-Bild enthält dabei Strukturen, deren Bewegung im Allgemeinen in ihrer Geschwindigkeit und/oder Amplitude deutlich kleiner sind als die im nicht-anatomischen 3D-Bild enthaltenen, in der Regel aktiv geführten, Strukturen. Da die darin enthaltene Patientenanatomie jedoch nur aus einer vergleichsweise hohen Anzahl an 2D-Aktualisierungsbilderm korrekt in ein 3D-Bild überführt werden kann, ist es möglich, die 3D-Bilder aus vergleichsweise vielen, jedoch über einen längeren Zeitpunkt aufgenommenen 2D-Aktualisierungsbildern zu berechnen.

Die Erfinder haben somit erkannt, dass eine 3D-Echtzeitdarstellung von Interventionsmaterialen in einer Patientenanatomie lediglich die 3D-Echtzeitrekonstruktionen der Interventionsmaterialien aus wenigen Projektionen voraussetzt, während die 3D-Rekonstruktion der Patientenanatomie viele 2D-Aktualisierungsbilder benötigt, die aber über einen längeren Zeitraum aufgenommen werden können. Die Erfinder haben weiterhin erkannt, dass die beschriebene Auftrennung der entsprechenden 3D-Rekonstruktionen, insbesondere dann, wenn es sich bei den 2D-Aktualisierungsbildern um 2D-Röntgenprojektionen handelt, eine dosisreduzierte Bildgebung ermöglicht, da die Aufnahme der 2D-Röntgenprojektionen mit einer vergleichsweise niedrigen zeitlichen Rate erfolgen kann, beispielsweise 5 pro Sekunde oder 10 pro Sekunde.

Erfindungsgemäß ist es vorgesehen, dass nach der Rekonstruktion des anatomischen 3D-Bildes eine Registrierung des anatomischen 3D-Bildes mit dem ersten 3D-Bild unter Bestimmung einer Koordinatentransformation erfolgt, d. h. das anatomische 3D-Bild wird in das Koordinatensystem des ersten 3D-Bildes überführt oder das erste 3D-Bild wird in das Koordinatensystem des anatomischen 3D-Bildes überführt. Vorteilhaft an einer solchen Vorgehensweise kann sein, dass aufgetretene Änderungen zwischen dem anatomischen 3D-Bild und dem ersten 3D-Bild, welches die wenigstens eine anatomische Struktur, insbesondere ein durch Kontrastmittel sichtbar gemachtes Gefäß, enthält, zu erfassen. Diese Änderungen können insbesondere dadurch bedingt sein, dass die Aufnahme des ersten 3D-Bildes mit einem anderen Bildgebungsgerät erfolgen kann als das für die Aufnahme der 2D-Aktualisierungsbilder verwendete. Da Unterschiede zwischen dem anatomischen 3D-Bild und dem nicht-anatomischen 3D-Bild im Allgemeinen vergleichsweise gering sind und sich im Wesentlichen auf die Bewegungen der nicht-anatomischen Strukturen, beispielsweise eines Führungsdrahts oder eines Katheters, beschränken, gilt die bestimmte Koordinatentransformation nun auch zwischen dem ersten 3D-Bild und dem nicht-anatomischen 3D-Bild. Es ist somit möglich, das nicht-anatomische 3D-Bild, beispielsweise einen Führungsdraht oder einen Katheter innerhalb eines Gefäßes, lagerichtig zum ersten 3D-Bild, und damit zu der wenigstens einen anatomischen Struktur, darzustellen. Diese anatomische Struktur ist dabei lediglich im ersten 3D-Bild sichtbar, beispielsweise durch die einmalige Verwendung eines Kontrastmittels innerhalb eines Blutgefäßes, nicht jedoch im anatomischen 3D-Bild, welches die anatomische Struktur im Allgemeinen nicht zeigt, da beispielsweise keine ständige Kontrastmittelgabe erfolgen kann.

Abschließend erfolgt somit eine Erstellung eines Navigationsvolumens aus dem wenigstens einen Anatomiemodell, dem nicht-anatomischen 3D-Bild sowie in alternativen Ausgestaltungen des erfindungsgemäßen Verfahrens zusätzlich dem anatomischen 3D-Bild unter Verwendung der bestimmten Koordinatentransformation. Somit werden im Navigationsvolumen sowohl die nicht-anatomischen Strukturen aus dem nicht-anatomischen 3D-Bild als auch das extrahierte Anatomiemodell, sowie in alternativen Ausgestaltungen des erfindungsgemäßen Verfahrens zusätzlich die anatomischen Strukturen aus dem anatomischen 3D-Bild zueinander lagerichtig dargestellt. Die Koordinatentransformation, welche aus dem anatomischen 3D-Bild und dem ersten 3D-Bild bestimmt wurde, ist vorzugsweise deformierbar. Bei der Bestimmung der Koordinatentransformation werden vorzugsweise, falls vorhanden, die kontrastmittelhaltigen Bereiche des ersten 3D-Bildes nicht berücksichtigt, da sie die Bestimmung der Koordinatentransformation verfälschen können. Die Bestimmung der vorzugsweise deformierbaren Koordinatentransformation zwischen dem ersten 3D-Bild und dem anatomischen-3D-Bild kann als Bildregistrierung aufgefasst werden.

In alternativen Ausgestaltungen des erfindungsgemäßen Verfahrens kann es sich bei der ersten Teilmenge der 2D-Aktualisierungsbilder, aus der das nicht-anatomische 3D-Bild berechnet wird, um die zuletzt aufgenommenen 2D-Aktualisierungsbilder handeln, beispielsweise um die letzten zwei oder drei 2D-Aktualisierungsbilder, wobei die Anzahl der 2D-Aktualisierungsbilder der ersten Teilmenge in einem Organprogramm des medizinischen Bildgebungsgerätes von einem Anwender eingestellt werden kann. Beispielsweise kann in diesem Organprogramm auch eingestellt werden, dass alle aufgenommenen 2D-Aktualisierungsbilder für die Rekonstruktion des nicht-anatomischen 3D-Bildes verwendet werden.

In alternativen Ausgestaltungen ist es vorgesehen, dass, wenn keine nicht-anatomische Struktur in einem 2D-Aktualisierungsbild der ersten Teilmenge vorhanden ist, erfindungsgemäß ebenfalls das nicht-anatomische 3D-Bild rekonstruiert werden kann, wobei dieses nicht-anatomische 3D-Bild leer ist, also beispielsweise nur Nullen enthält.

Ferner können in alternativen Ausgestaltungen des erfindungsgemäßen Verfahrens die nicht-anatomischen 3D-Bilder und/oder die anatomischen 3D-Bilder insbesondere mit einer zeitlichen Rate rekonstruiert werden, wobei die zeitliche Rate der Rekonstruktion der anatomischen 3D-Bilder insbesondere der Aufnahmerate der 2D-Aktualisierungsbilder entspricht. Die Rekonstruktion mit einer zeitlichen Rate ermöglicht es, dass das nicht-anatomische 3D-Bild beispielsweise in Echtzeit rekonstruiert und im Navigationsvolumen angezeigt werden kann. Die Rekonstruktion der nicht-anatomischen 3D-Bilder in Echtzeit ist insbesondere deshalb realisierbar, weil nicht-anatomische Strukturen, insbesondere Interventionsmaterialien, beispielsweise zylindrische Strukturen, beispielsweise Führungsdrähte, insbesondere entlang deren Axialachse, in der Regel eine hohe Symmetrie aufweisen, und weil die nicht-anatomischen Strukturen vorzugsweise nur einen geringen Anteil des abzubildenden Volumens ausfüllen und daher diese nicht-anatomischen Strukturen aus nur wenigen 2D-Aktualisierungsbildern rekonstruiert werden können.

In alternativen Ausgestaltungen des erfindungsgemäßen Verfahrens kann bei der Aufnahme eines neuen 2D-Aktualisierungsbildes dieses zur zweiten Teilmenge der zur Verfügung gestellten 2D-Aktualisierungsbilder hinzugefügt werden und das anatomische 3D-Bild wenigstens teilweise neu rekonstruiert werden, wobei zeitlich ältere 2D-Aktualisierungsbilder für die erneute Rekonstruktion wiederverwendet werden können. Dies kann als überlappende Rekonstruktion bezeichnet werden. Eine Möglichkeit, den Rechenaufwand für das erfindungsgemäße Verfahren hierfür gering zu halten, besteht darin, im Rahmen der Rekonstruktion lediglich wenigstens ein neu hinzugekommenes 2D-Aktualisierungsbild auf das anatomische 3D-Bild aufzuaddieren, beispielsweise mittels gefilterter Rückprojektion, und das älteste 2D-Aktualisierungsbild, welches aus einem Zeitfenster herausfällt, durch Subtraktion aus dem anatomischen 3D-Bild zu entfernen, beispielsweise durch Subtraktion seiner gefilterten Rückprojektion. Die Anzahl der 2D-Aktualisierungsbilder in der zweiten Teilmenge, kann dabei in einem Organprogramm durch den Anwender eingestellt werden. Ferner besteht die Möglichkeit, ggf. vorliegende Informationen über die Bewegung des Patienten, beispielsweise Atembewegung, Herzbewegung, Patiententischbewegung, in die Rekonstruktion des anatomischen 3D-Bildes einfließen zu lassen.

In alternativen Ausgestaltungen kann das erfindungsgemäße Verfahren, insbesondere die Berechnung des nicht-anatomischen 3D-Bildes aus den Teil-Rekonstruktionen mit einem Maschinenlernverfahren, insbesondere einem neuronalen Netz, beispielweise einem Convolutional Neural Network, erfolgen. Vorteilhaft an diesen Ausgestaltungen kann sein, dass nur wenige 2D-Aktualisierungsbilder benötigt werden, um ein nicht-anatomisches 3D-Bild zu rekonstruieren. Erfindungsgemäß können wenige 2D-Aktualisierungsbilder auch als ein 2D-Aktualisierungsbild verstanden werden.

Das Wesen eines Maschinenlernverfahren besteht darin, Inputdaten gemäß einer bestimmten Transformation zu Outputdaten zu transformieren. Um das Maschinenlernverfahren in die Lage zu versetzen, die gewünschte Transformation durchzuführen, können seine freien Parameter in einem iterativen Prozess (Training genannt) geeignet eingestellt bzw. gelernt werden. Vorzugsweise geschieht dies in einem überwachten Training, d.h. dem Maschinenlernverfahren wird eine Vielzahl von Paaren (Trainingspaare genannt), jeweils bestehend aus möglichem Inputdatum einerseits und, gemäß der zu erlernenden Transformation zugehörigen, Outputdatum andererseits, präsentiert. Hierbei werden die freien Parameter des Netzes iterativ so angepasst, dass der Wert einer Funktion, vorzugsweise einer Kostenfunktion, welche die Abweichung zwischen den tatsächlichen Outputdaten und den, gemäß der zu erlernenden Transformation, gewünschten Outputdaten misst, minimiert wird. Die Trainingspaare werden vorzugsweise durch realistische Simulation erstellt, da somit eine hohe Anzahl an Trainingspaaren erzeugt werden kann. Vorteilhaft hierbei kann sein, dass die Genauigkeit des Maschinenlernverfahrens mit der Anzahl an Trainingspaaren steigt. Vorzugsweise wird mittels realistischer Simulation von nicht-anatomischen Strukturen und realistischer Simulation von daraus erhaltenen Teil-Rekonstruktionen, welche diese nicht-anatomischen Strukturen beinhalten, eine hohe Anzahl an Trainingspaaren erzeugt, wobei jedes Trainingspaar aus einer Menge von Teil-Rekonstruktionen einerseits und dem zugehörigen nicht-anatomischen 3D-Bild andererseits besteht.

In alternativen Ausgestaltungen des erfindungsgemäßen Verfahrens ist es vorgesehen, dass auftretende Bewegungen des Patienten, beispielsweise Atembewegung, Herzbewegung, Patiententischbewegung berücksichtigt werden. Diese Berücksichtigung der Bewegungen kann dabei implizit erfolgen, indem die Teil-Rekonstruktionen separate Eingangskanäle für das erfindungsgemäße Verfahren darstellen. Sie kann jedoch auch explizit erfolgen, indem dem erfindungsgemäßen Verfahren ein solches Verfahren vorangestellt wird, welches zunächst separat eine Bewegungskorrektur durchführt., beispielsweise können Translation, Rotation und/oder Deformation korrigiert werden. Bei dem Verfahren kann es sich beispielsweise um ein Maschinenlernverfahren, insbesondere ein neuronales Netz, beispielweise ein Convolutional Neural Network handeln.

In alternativen Ausgestaltungen kann das erfindungsgemäße Verfahren, insbesondere die Extraktion der anatomischen und nicht-anatomischen Strukturen aus den 2D-Aktualisierungsbildern, mit wenigstens einem Maschinenlernverfahren, insbesondere einem neuronalen Netz, beispielweise einem Convolutional Neural Network, erfolgen. Vorteilhaft an diesen Ausgestaltungen kann sein, dass Maschinenlernverfahren Transformationen, die wie die im vorangegangenen Abschnitt offenbarte Extraktion einer Segmentierung ähnlich sind, mit hoher Genauigkeit ausführen können.

In alternativen Ausgestaltungen des erfindungsgemäßen Verfahrens ist es vorgesehen, dass die Berechnung des nicht-anatomischen 3D-Bildes aus den 2D-Aktualisierungsbildern durch ein einziges Maschinenlernverfahren durchgeführt wird, beispielsweise durch ein neuronales Netz, das darauf trainiert wurde, 2D-Aktualisierungsbilder zunächst in andere 2D-Bilder zu überführen, beispielsweise in extrahierte anatomische Strukturen oder nicht-anatomische Strukturen, wobei diese anderen 2D-Bilder anschließend im selben Maschinenlernverfahren zu einem artefaktfreien nicht-anatomischen 3D-Bild rekonstruiert werden.

In alternativen Ausgestaltungen des erfindungsgemäßen Verfahrens kann die Extraktion von nicht-anatomischen Strukturen derart erfolgen, dass nur bestimmte nicht-anatomische Strukturen extrahiert werden, beispielsweise solche, die zu einer bestimmten Objektklasse gehören, oder zu einer bestimmten Kombination mehrerer Objektklassen. Nachfolgend werden also nur solche nicht-anatomische Strukturen zu nicht-anatomischen 3D-Bildern rekonstruiert, die diese bestimmten nicht-anatomischen Strukturen zeigen, die also zu der bestimmten Objektklasse gehören, oder der bestimmten Kombination mehrerer Objektklassen. Wird für die Extraktion ein Maschinenlernverfahren verwendet, wird dieses entsprechend nicht nur auf die Extraktion, sondern auch auf die Unterscheidung solcher nicht-anatomischer Strukturen trainiert. Vorteilhaft an solchen alternativen Ausgestaltungen kann es sein, beispielsweise bei einer Navigation eines Führungsdrahtes in einem Patienten nur diesen Führungsdraht zu extrahieren und zu rekonstruieren. Weitere, beispielsweise schon vorhandene und insbesondere zu anderen Objektklassen gehörige nicht-anatomische Strukturen, beispielsweise Stents oder orthopädische Implantate aus vergangenen Eingriffen, oder nicht-eingriffsspezifische nicht-anatomische Strukturen, beispielsweise der Patiententisch, welche dann nicht von Interesse sind, werden dann weder extrahiert noch rekonstruiert, sodass die dem behandelnden Arzt angezeigte Darstellung auf das Wesentliche reduziert wird.

In alternativen Ausgestaltungen des erfindungsgemäßen Verfahrens kann die Extraktion von nicht-anatomischen Strukturen derart erfolgen, dass aus jedem 2D-Aktualisierungsbild mehrere separate Extraktionen berechnet werden, wobei jede der separaten Extraktionen nur eine Teilmenge der nicht-anatomischen Strukturen zeigt, beispielsweise nur solche, die zu einer bestimmten Objektklasse gehören, oder zu einer bestimmten Kombination mehrerer Objektklassen. Im Anschluss werden die Extraktionen separat zu nicht-anatomischen 3D-Bildern verarbeitet, wobei die so berechneten separaten nicht-anatomischen 3D-Bilder nur die entsprechende Teilmenge der nicht-anatomischen Strukturen enthalten. Anschließend werden die separaten nicht-anatomischen 3D-Bilder zu einem einzigen nicht-anatomischen 3D-Bild kombiniert, beispielsweise durch Addition voxelbasierter separater nicht-anatomischer 3D-Bilder. Vorteilhaft an solchen alternativen Ausgestaltungen kann es sein, dass ein anatomisches 3D-Bild, das durch separate 3D-Rekonstruktionen von Teilmengen der nicht-anatomischen Strukturen und anschließender Kombination erzeugt wurde, genauer sein kann als ein auf einmal rekonstruiertes anatomisches 3D-Bild, denn das Rekonstruktionsproblem ist im Allgemeinen umso einfacher, je geringer der Anteil der nicht-anatomischen Strukturen am abzubildenden Volumen ist.

Ein weiterer Aspekt der Erfindung betrifft ein medizinisches Bildgebungsgerät, insbesondere ein gantrybasiertes System, zur Durchführung einer bildgebenden Untersuchung und Darstellung von nicht-anatomischen Strukturen, beinhaltend die folgenden Bestandteile:
- eine Zurverfügungstellungseinheit, zum Zurverfügungstellen eines ersten 3D-Bildes, wobei das erste 3D-Bild wenigstens eine anatomische Struktur enthält;
- wenigstens zwei Bildketten, wobei die Bildketten zum Aufnehmen von 2D-Bildern, insbesondere 2D-Aktualisierungsbildern und/oder 3D-Bildern ausgebildet sind;
- eine Recheneinheit,
   wobei die Recheneinheit zur Extraktion eines Anatomiemodells aus der wenigstens einen anatomischen Struktur ausgebildet ist,
   wobei die Recheneinheit zur Extraktion von anatomischen und nicht-anatomischen Strukturen aus den 2D-Aktualisierungsbildern ausgebildet ist,
   wobei die Recheneinheit zur Berechnung von Teil-Rekonstruktionen aus der Extraktion nicht-anatomischer Strukturen ausgebildet ist,
   wobei die Recheneinheit zur Berechnung eines nicht-anatomischen 3D-Bildes aus wenigstens zwei Teil-Rekonstruktionen ausgebildet ist,
   wobei die Recheneinheit zur Berechnung eines anatomischen 3D-Bildes aus der Extraktion anatomischer Strukturen ausgebildet ist,
   und wobei die Recheneinheit zur Erstellung eines Navigationsvolumens aus dem Anatomiemodell und dem nicht-anatomischen 3D-Bild unter Bestimmung einer Koordinatentransformation ausgebildet ist, wobei die Koordinatentransformation zur lagerichtigen Positionierung des Anatomiemodells im Navigationsvolumen ausgebildet ist;
- eine Darstellungseinheit, auf welcher die Darstellung des Navigationsvolumens erfolgt.

Die erfindungsgemäße Vorrichtung beinhaltet eine Zurverfügungstellungseinheit, vorzugsweise eine Speichereinheit, beispielsweise ein Memorystick, eine Festplatte oder ein sonstiger transportabler oder fest eingebauter Datenträger. Die Zurverfügungstellungseinheit kann auch als eine Verbindung zu einer Speichereinheit, welche an einem Netzwerk angebunden ist, zu welchem das medizinische Bildgebungsgerät einen Zugang hat, verstanden werden, um somit ein erstes 3D-Bild, wobei das erste 3D-Bild wenigstens eine anatomische Struktur enthält, zur Verfügung zu stellen.

Die erfindungsgemäße Vorrichtung beinhaltet dabei wenigstens zwei Bildketten, wobei die erfindungsgemäße Vorrichtung auch beispielweise drei, vier oder mehr Bildketten umfassen kann. Eine Bildkette beinhaltet dabei einen Röntgengenerator zur Erzeugung von Röntgenstrahlung, beispielsweise ein drehanodenbasierter Generator und eine Empfangseinheit zum Aufnehmen von Röntgenstrahlung, beispielsweise ein Flachdetektor, wobei die Bildketten zum Aufnehmen von 2D-Bildern, insbesondere 2D-Aktualisierungsbildern, und/oder 3D-Bildern ausgebildet sind.

Vorteilhaft bei der Verwendung von wenigstens zwei Bildketten kann sein, dass gleichzeitig, pseudo-gleichzeitig oder direkt aufeinanderfolgend mehrere 2D-Aktualisierungsbilder aufgenommen werden können. Vorzugsweise beinhaltet das medizinische Bildgebungsgerät zwei Bildketten, da eine höhere Anzahl von Bildketten die Komplexität, die Kosten und die Ausfallwahrscheinlichkeit des medizinischen Bildgebungsgerätes erhöht. Die Aufnahme von 2D-Aktualisierungsbildern erfolgt vorzugsweise in Tupeln, wobei sich das gantrybasierte System zwischen der Aufnahme von 2D-Aktualisierungsbildern eines Tupels vorzugsweise nicht oder näherungsweise nicht weitergedreht hat. Dagegen kann sich das gantrybasierte System zwischen der Aufnahme von Tupeln vorzugsweise um einen Mindestwinkel von beispielsweise wenigstens 5° oder 20° weiterdrehen. Bei einer Rotation des gantrybasierten Systems liegt vorzugsweise eine niedrige Rotationsgeschwindigkeit vor, da bei einer zu hohen Rotationsgeschwindigkeit Bewegungsunschärfe auftreten kann.

Ferner beinhaltet die erfindungsgemäße Vorrichtung eine Recheneinheit, wobei diese Recheneinheit eine Grafikprozessoreinheit (Graphics Processing Unit, GPU) oder eine anderweitige Prozessoreinheit, beispielsweise eine Central Processing Unit (CPU) sein kann, wobei im Falle einer GPU diese dazu verwendet werden kann, massiv- bzw. hochparallele Rechenoperationen auszuführen. Eine Recheneinheit kann dabei auch aus mehreren Recheneinheiten bestehen, welche in ihrer Gesamtheit wieder als eine erfindungsgemäße Recheneinheit aufgefasst werden können. Ferner extrahiert diese Recheneinheit ein Anatomiemodell aus der anatomischen Struktur des ersten 3D-Bildes. Die Recheneinheit ist ebenfalls dazu ausgebildet, anatomische und nicht-anatomische Strukturen aus den wenigstens zwei 2D-Aktualisierungsbildern zu extrahieren. Ferner rekonstruiert die Recheneinheit wenigstens zwei Teil-Rekonstruktionen aus den Extraktionen der nicht-anatomischen Strukturen der ersten Teilmenge der 2D-Aktualisierungsbilder und rekonstruiert ebenfalls ein anatomisches 3D-Bild aus der Extraktion anatomischer Strukturen der zweiten Teilmenge von 2D-Aktualisierungsbildern. Die Recheneinheit ist ebenfalls zur Berechnung eines nicht-anatomischen 3D-Bildes aus wenigstens zwei Teil-Rekonstruktionen ausgebildet. Die Recheneinheit dient weiterhin der Erstellung eines Navigationsvolumens aus dem Anatomiemodell, dem nicht-anatomischen 3D-Bild und optional dem anatomischen 3D-Bild unter Bestimmung einer Koordinatentransformation, wobei die Koordinatentransformation zur lagerichtigen Positionierung des Anatomiemodells, des nicht-anatomischen 3D-Bildes und optional des anatomischen 3D-Bildes im Navigationsvolumen ausgebildet ist.

Weiterhin beinhaltet die erfindungsgemäße Vorrichtung eine Darstellungseinheit, auf welcher die Darstellung des Navigationsvolumens erfolgt, sowie jeweils optional des nicht-anatomischen 3D-Bildes, des anatomischen 3D-Bildes, des ersten 3D-Bildes und/oder des Anatomiemodells. Die Darstellungseinheit kann darüber hinaus dahingehend ausgebildet sein, die 2D-Aktualisierungsbilder anzuzeigen. In weiteren vorteilhaften Ausgestaltungen des erfindungsgemäßen Verfahrens kann die Darstellungseinheit darüber hinaus, insbesondere bei einem Stillstand der Gantry, konventionelle Röntgenbilder darstellen, beispielsweise Fluoroskopien, sowie mittels der Recheneinheit durchgeführte Nachbearbeitungen solcher Röntgenbilder, beispielsweise mittels eines Frequenzfilters. In weiteren erfindungsgemäßen Ausgestaltungen ist vorgesehen, Bilder darzustellen, die mittels eines Subtraktionsverfahrens auf der Recheneinheit berechnet wurden, beispielsweise eine DSA.

Nachfolgend wird die Erfindung anhand der Abbildungen näher erläutert.
Fig. 1 zeigt schematisch einen Ablauf des erfindungsgemäßen Verfahrens.
Fig. 2 zeigt exemplarisch einen Ablaufplan des erfindungsgemäßen Verfahrens, bei welchem neue 2D-Aktualisierungsbilder hinzugefügt werden.
Fig. 3 zeigt exemplarisch einen Ablaufplan des erfindungsgemäßen Verfahrens, bei welchem neue 2D-Aktualisierungsbilder hinzugefügt werden und alte 2D-Aktualisierungsbilder durch diese ersetzt werden.
Fig. 4 zeigt schematisch eine Bildgebungsvorrichtung, welche das erfindungsgemäße Verfahren ausführen kann.

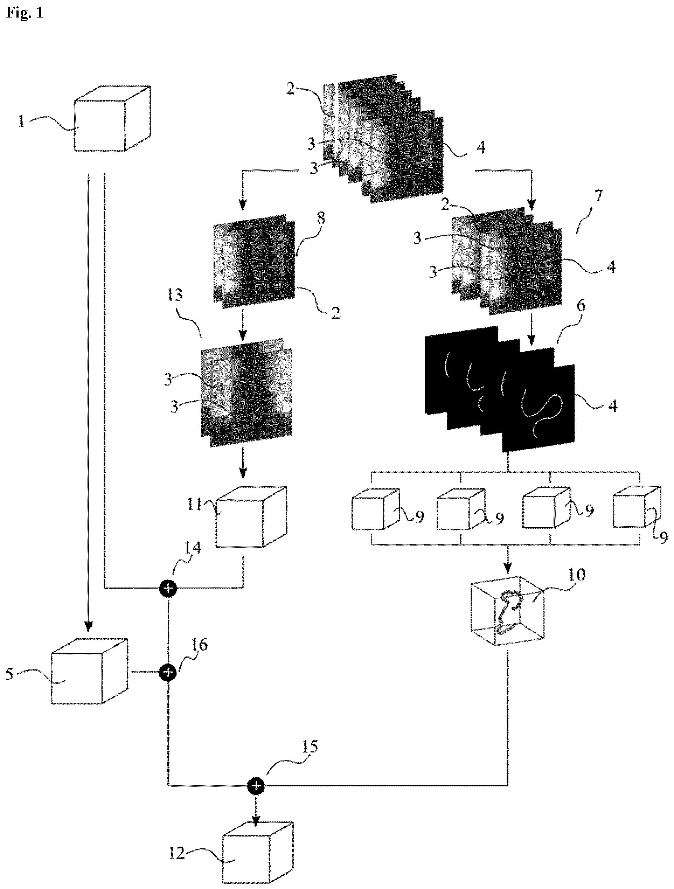

Fig. 1 zeigt einen exemplarischen Ablauf des erfindungsgemäßen Verfahrens. Zunächst wird dem erfindungsgemäßen Verfahren ein erstes 3D-Bild 1 (3D-Volumen) zur Verfügung gestellt. Dieses erste 3D-Bild 1 kann während oder vor einem interventionellen Eingriff mittels einer 3D-Aufnahme aufgenommen werden. Aus diesem ersten 3D-Bild 1 wird ferner ein Anatomiemodell 5 extrahiert.

Anschließend erfolgt gemäß dem erfindungsgemäßen Verfahren eine Zurverfügungstellung von wenigstens zwei 2D-Aktualisierungsbildern 2, wobei die 2D-Aktualisierungsbilder 2 wenigstens teilweise anatomische Strukturen 3 und/oder wenigstens teilweise nicht-anatomische Strukturen 4 eines Untersuchungsbereiches enthalten. Die wenigstens teilweise anatomischen Strukturen 3 sind dabei Teile der Knochenstrukturen und/oder Teile der Gefäßstrukturen und/oder Teile der Weichteilgewebe. In Fig. 1 ist als nicht-anatomische Struktur 4 ein Führungsdraht in einem Untersuchungsbereich vorhanden. Ferner wurden die wenigstens zwei 2D-Aktualisierungsbilder 2 in einem Zeitintervall aufgenommen.

Im Anschluss an die Zurverfügungstellung der wenigstens zwei 2D-Aktualisierungsbilder 2 erfolgt eine Extraktion 6 von nicht-anatomischen Strukturen 4 aus einer ersten Teilmenge 7 der wenigstens zwei 2D-Aktualisierungsbilder 2.

Basierend auf diesen Extraktionen 6 der nicht-anatomischen Strukturen 4 aus der ersten Teilmenge 7 der 2D-Aktualisierungsbilder 2 wird eine Berechnung eines nicht-anatomischen 3D-Bildes 10 aus mehreren Teil-Rekonstruktionen 9 durchgeführt. In Fig. 1 geschieht dies aus vier Teil-Rekonstruktionen 9. Die verschiedenen Teil-Rekonstruktionen 9 gehen dabei vorzugsweise aus 2D-Aktualisierungsbildern hervor, welche zu unterschiedlichen Zeitpunkten aufgenommen wurden und können somit, aufgrund möglicher Bewegungen des Patienten und/oder der nicht-anatomischen Strukturen, verschiedene Zustände, Positionen und Orientierungen der nicht-anatomischen Strukturen 4 darstellen.

Neben der Extraktion 6 der nicht-anatomischen Strukturen 4 erfolgt ebenfalls eine Extraktion 13 der anatomischen Strukturen 3 aus einer zweiten Teilmenge 8 der 2D-Aktualisierungsbilder 2, wobei die zweite Teilmenge 8 sich wenigstens in einem 2D-Aktualisierungsbild 2 von der ersten Teilmenge 7 unterscheidet, so dass beide Teilmengen (7,8) nicht identisch sind. Aus der Extraktion 13 anatomischer Strukturen 3 wird ein anatomisches 3D-Bild 11 berechnet.

Erfindungsgemäß ist es vorgesehen, dass nach der Rekonstruktion des anatomischen 3D-Bildes 11 eine Registrierung des anatomischen 3D-Bildes 11 mit dem ersten 3D-Bild 1 unter Bestimmung einer Koordinatentransformation 14 erfolgt. Anschließend erfolgt eine Anwendung 16 der so erhaltenen Koordinatentransfromation 14 auf das Anatomiemodell 5. Die Koordinatentransformation, welche aus dem anatomischen 3D-Bild 11 und dem ersten 3D-Bild 1 bestimmt wurde, ist vorzugsweise deformierbar. Bei der Bestimmung der Koordinatentransformation 14 werden vorzugsweise, falls vorhanden, die kontrastmittelhaltigen Bereiche des ersten 3D-Bildes 1 nicht berücksichtigt, da sie die Bestimmung der Koordinatentransformation verfälschen können.

Abschließend wird ein Navigationsvolumen 12 erstellt 15 aus dem wenigstens einen Anatomiemodell 5 und dem nicht-anatomischen 3D-Bild 10. Somit werden im Navigationsvolumen 12 sowohl die nicht-anatomischen Strukturen 4 aus dem nicht-anatomischen 3D-Bild 10, als auch das extrahierte Anatomiemodell 5 zueinander lagerichtig dargestellt.

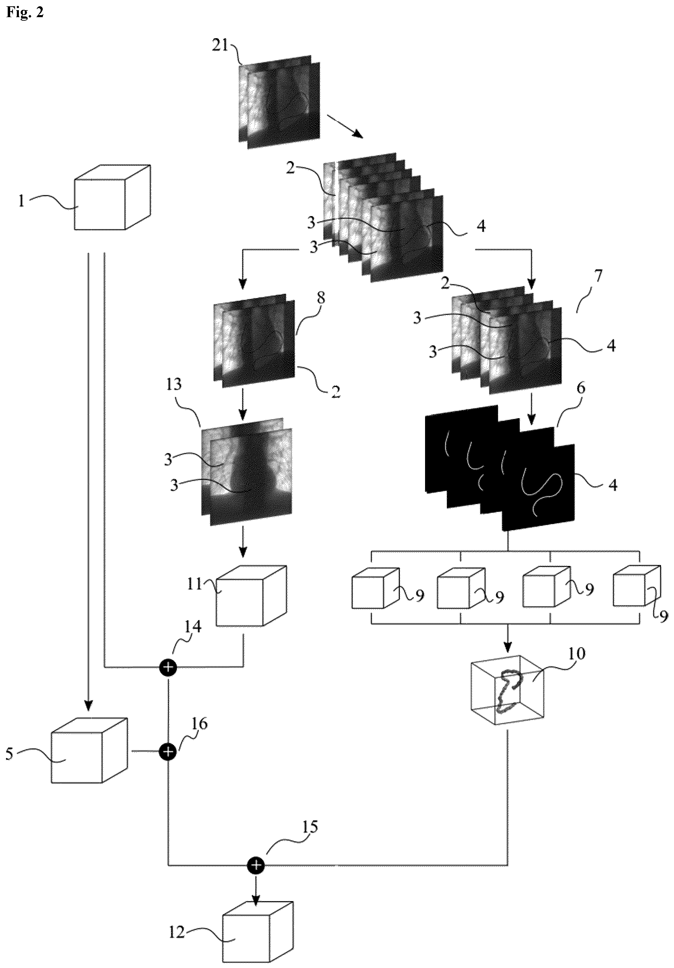

Fig. 2 zeigt einen Ablauf des erfindungsgemäßen Verfahrens, bei welchem nach Aufnahme von zwei neuen 2D-Aktualisierungsbildern 21 diese zur ersten Teilmenge 7 und zur zweiten Teilmenge 8 der zur Verfügung gestellten 2D-Aktualisierungsbilder 2 hinzugefügt werden und das anatomische 3D-Bild 11 wenigstens teilweise neu rekonstruiert wird, wobei alle zeitlich älteren 2D-Aktualisierungsbilder 2 für die erneute Rekonstruktion des anatomischen 3D-Bildes 11 wiederverwendet werden können. Ferner wird mittels der neuen 2D-Aktualisierungsbilder 21 das nicht-anatomische 3D-Bild 10 neu rekonstruiert. Der hier beschriebene Ablauf des erfindungsgemäßen Verfahrens ist insbesondere dann, beispielsweise zu Beginn der Bildgebung, vorteilhaft, wenn noch nicht genügend 2D-Aktualisierungsbilder 2 in der zweiten Teilmenge 8 vorliegen, um aus ihnen ein artefaktarmes anatomisches 3D-Bild 11 zu rekonstruieren. Durch die Erhöhung der Zahl der 2D-Aktualisierungsbilder 2 in der zweiten Teilmenge 8 wird vorzugsweise eine höhere Bildqualität erreicht.

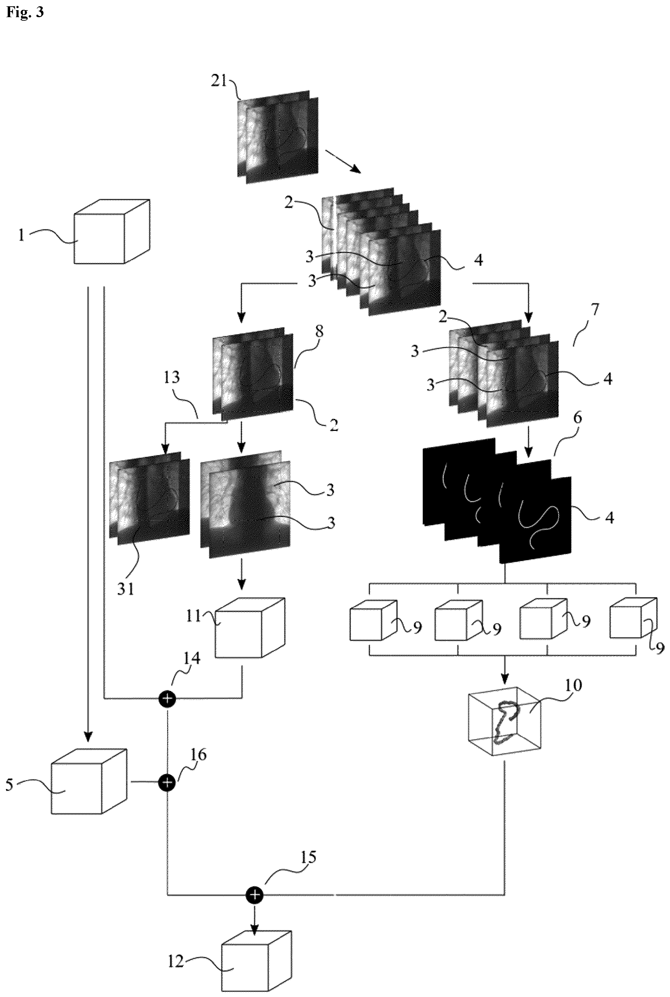

Fig. 3 zeigt eine Ausführung des erfindungsgemäßen Verfahrens, bei welcher zwei neu aufgenommene 2D-Aktualisierungsbilder 21 zur zweiten Teilmenge 8 hinzugefügt werden und die ältesten zwei 2D-Aktualisierungsbilder 31 aus der zweiten Teilmenge entfernt werden. Aus der so veränderten zweiten Teilmenge wird dann erneut ein anatomisches 3D-Bild 11 rekonstruiert. Dies kann als überlappende Rekonstruktion bezeichnet werden. Vorteilhaft an dieser Ausführung ist, dass das so veränderte anatomische 3D-Bild nun aktuellere Informationen über die anatomischen Strukturen 3, welche sich beispielsweise durch Atem-, Herz- oder Patiententischbewegung verändert haben können, enthält.

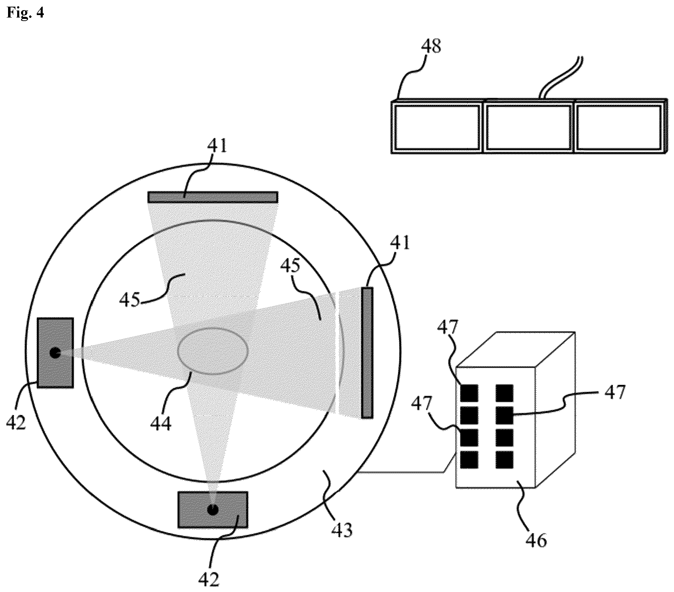

Fig. 4 zeigt schematisch eine Bildgebungsvorrichtung, welche dazu ausgebildet ist, das erfindungsgemäße Verfahren auszuführen. Dabei beinhaltet die Bildgebungsvorrichtung eine rotierend gelagerte Gantry 43, wobei auf dieser rotierend gelagerten Gantry zwei Röntgenstrahlengeneratoren 42 angebracht sind sowie den Röntgenstrahlengeneratoren direkt gegenüberliegend röntgenstrahlenabsorbierende Flachdetektoren 41 angebracht sind. Die Einheit aus einem Röntgenstrahlengenerator und dem diesem gegenüberliegend angeordneten Flachdetektor 41 kann als Bildkette bezeichnet werden. Diese Bildketten können dazu genutzt werden, ein erstes 3D-Bild und 2D-Aktualisierungsbilder für das erfindungsgemäße Verfahren zur Verfügung zu stellen. Die verschiedenen Bildketten können die 2D-Aktualisierungsbilder dabei gleichzeitig bzw. pseudo-gleichzeitig aufnehmen oder auch mit einem zeitlichen Versatz. Der mittels Röntgenstrahlen 45 zu untersuchende Bereich 44 kann dabei anatomische und nicht-anatomische Strukturen enthalten. Die für die Verarbeitung der 2D-Aktualisierungsbilder und des ersten 3D-Bildes benötigten Recheneinheiten 47 sind in Fig. 4 in einer ausgelagerten Verarbeitungseinheit 46 integriert. Alternativ kann diese Verarbeitungseinheit 46 bzw. die Recheneinheiten 47 direkt in die Bildgebungsvorrichtung integriert sein. Auf einer externen Darstellungseinheit 48 kann das erfindungsgemäße Navigationsvolumen dargestellt werden. Alternativ kann die Anzeigeeinheit auch direkt an der Bildgebungsvorrichtung angebracht sein.

### Bezugszeichenliste

- 1: erstes 3D-Bild
- 2: 2D-Aktualisierungsbild
- 3: anatomische Strukturen
- 4: nicht-anatomische Struktur
- 5: Anatomiemodell
- 6: Extraktion der nicht-anatomischen Strukturen
- 7: erste Teilmenge
- 8: zweite Teilmenge
- 9: Teil-Rekonstruktion
- 10: nicht-anatomisches 3D-Bild
- 11: anatomisches 3D-Bild
- 12: Navigationsvolumen
- 13: Extraktion der anatomischen Strukturen
- 14: Bestimmung der Koordinatenttransformation
- 15: Erstellung Navigationsvolumen
- 16: Anwendung der Koordinatentransformation

- 21: neu aufgenommenes 2D-Aktualisierungsbild
- 31: entfernte 2D-Aktualisierungsbilder

- 41: Röntgendetektor
- 42: Röntgengenerator
- 43: rotierbar gelagerte Gantry
- 44: Untersuchungsbereich
- 45: Röntgenstrahlen
- 46: Verarbeitungseinheit
- 47: Recheneinheit
- 48: Darstellungseinheit

## Patentansprüche

1. Verfahren zum Betreiben eines medizinischen Bildgebungsgerätes zur lagerichtigen Darstellung von nicht-anatomischen Strukturen während einer bildgebenden Untersuchung, beinhaltend die folgenden Verfahrensschritte:
a.) Zurverfügungstellung eines ersten 3D-Bildes (1), welches wenigstens eine anatomische Struktur (3) enthält;
b.) Extraktion wenigstens eines Anatomiemodells (5) aus der wenigstens einen anatomischen Struktur (3) des ersten 3D-Bildes (1);
c.) Zurverfügungstellung wenigstens zweier 2D-Aktualisierungsbilder (2) mittels des medizinischen Bildgebungsgerätes, wobei wenigstens zwei 2D-Aktualisierungsbilder (2) zu unterschiedlichen Zeitpunkten aufgenommen wurden;
d.) Extraktion (6) nicht-anatomischer Strukturen (4) aus einer ersten Teilmenge (7) der 2D-Aktualisierungsbilder (2);
e.) Extraktion (13) anatomischer Strukturen (3) aus einer zweiten Teilmenge (8) der 2D-Aktualisierungsbilder (2);
f.) Berechnen eines nicht-anatomischen 3D-Bildes (10) aus wenigstens zwei Teil-Rekonstruktionen (9) aus der ersten Teilmenge (7), wobei die wenigstens zwei Teil-Rekonstruktionen (9) aus der Extraktion (6) der nicht-anatomischen Strukturen (4) berechnet werden und Artefakte der Teil-Rekonstruktionen mittels maschineller Lernverfahren entfernt werden;
g.) Rekonstruktion eines anatomischen 3D-Bildes (11) aus der Extraktion (13) der anatomischen Strukturen (3) aus der zweiten Teilmenge (8);
h.) Registrierung des anatomischen 3D-Bildes (11) mit dem ersten 3D-Bild (1) zur Bestimmung einer Koordinatentransformation (14);
i.) Erstellung eines Navigationsvolumens (12) aus dem wenigstens einem Anatomiemodell (5) und dem nicht-anatomischen 3D-Bild (10) unter Verwendung der bestimmten Koordinatentransformation (14).

2. Verfahren nach Anspruch 1, **dadurch gekennzeichnet, dass** das Navigationsvolumen (12) zusätzlich aus dem anatomischen 3D-Bild (11) unter Verwendung der bestimmten Koordinatentransformation (14) erstellt wird.

3. Verfahren nach Anspruch 1, **dadurch gekennzeichnet, dass** das erste 3D-Bild (1) mit einem Röntgengerät, insbesondere einem Röntgen-C-Bogen oder einem Computertomographen oder mit einem Magnetresonanztomographen aufgenommen wird.

4. Verfahren nach einem der vorhergehenden Ansprüche, **dadurch gekennzeichnet, dass** die 2D-Aktualisierungsbilder (2) mit einem Röntgengerät, insbesondere einem Röntgen-C-Bogen, oder einem Computertomographen aufgenommen werden.

5. Verfahren nach einem der vorhergehenden Ansprüche, **dadurch gekennzeichnet, dass** bei Abwesenheit nicht-anatomischer Strukturen (4) in einem 2D-Aktualisierungsbild (2) der ersten Teilmenge (7) das nicht-anatomische 3D-Bild (10) weiterhin rekonstruiert wird.

6. Verfahren nach einem der vorhergehenden Ansprüche, **dadurch gekennzeichnet, dass** neu aufgenommene 2D-Aktualisierungsbilder (21) zur ersten Teilmenge (7) und/oder zweiten Teilmenge (8) hinzugefügt werden und die nicht-anatomischen 3D-Bilder (10) und/oder die anatomischen 3D-Bilder (11), insbesondere mit einer zeitlichen Rate, rekonstruiert werden, wobei die zeitliche Rate der Rekonstruktion der nicht-anatomischen 3D-Bilder (10) und/oder der anatomischen 3D-Bilder (11) einer Funktion der Aufnahmerate der 2D-Aktualisierungsbilder (21) entspricht.

7. Verfahren nach einem der vorhergehenden Ansprüche, **dadurch gekennzeichnet, dass** bei Aufnahme eines neuen 2D-Aktualisierungsbildes (21) dieses zur zweiten Teilmenge (8) der zur Verfügung gestellten 2D-Aktualisieurngsbilder (2) hinzugefügt wird und das anatomische 3D-Bild (11) wenigstens teilweise neu rekonstruiert wird, wobei zeitlich ältere 2D-Aktualisierungsbilder für die erneute Rekonstruktion wiederverwendet oder entfernt werden.

8. Verfahren nach einem der vorhergehenden Ansprüche, **dadurch gekennzeichnet, dass** das erfindungsgemäße Verfahren, insbesondere die Berechnung des nicht-anatomischen 3D-Bildes (10) aus den Teil-Rekonstruktionen (9), mit einem Maschinenlernverfahren, insbesondere einem neuronalen Netz, erfolgt.

9. Verfahren nach einem der vorhergehenden Ansprüche, **dadurch gekennzeichnet, dass** auftretende Bewegungen des Patienten berücksichtigt werden.

10. Verfahren nach einem der vorhergehenden Ansprüche, **dadurch gekennzeichnet, dass** das Verfahren, insbesondere die Extraktion (6,13) der anatomischen (3) und/oder nicht-anatomischen (4) Strukturen aus den 2D-Aktualisierungsbildern (2), mit wenigstens einem Maschinenlernverfahren, insbesondere mit einem neuronalen Netz erfolgt.

11. Verfahren nach einem der vorhergehenden Ansprüche, **dadurch gekennzeichnet, dass** die Berechnung des nicht-anatomischen 3D-Bildes (10) aus den 2D-Aktualisierungsbildern (2) durch ein einziges Maschinenlernverfahren, insbesondere ein neuronales Netz, durchgeführt wird.

12. Verfahren nach einem der vorhergehenden Ansprüche, **dadurch gekennzeichnet, dass** nur solche nicht-anatomische Strukturen (4) extrahiert werden, die einer bestimmten Objektklasse oder einer Kombination bestimmter Objektklassen angehören.

13. Verfahren nach einem der vorhergehenden Ansprüche, **dadurch gekennzeichnet, dass** nicht-anatomische Strukturen (4), die einer bestimmten Objektklasse oder einer Kombination bestimmter Objektklassen angehören, separat rekonstruiert werden.

14. Verfahren nach einem der vorhergehenden Ansprüche, **dadurch gekennzeichnet, dass** es sich bei den nicht-anatomischen Strukturen (4) um einen Führungsdraht und/oder Katheter handelt.

15. Ein medizinisches Bildgebungsgerät zur Durchführung einer bildgebenden Untersuchung und Darstellung von nicht-anatomischen Strukturen, aufweisend:
• eine Zurverfügungstellungseinheit, zum Zurverfügungstellen eines ersten 3D-Bildes (1), wobei das erste 3D-Bild wenigstens eine anatomische Struktur enthält;
• wenigstens zwei Bildketten (41, 42), wobei die Bildketten zum Aufnehmen von 2D-Aktualisierungsbildern (2) ausgebildet sind;
• eine Recheneinheit (47),
• wobei die Recheneinheit (47) zu Folgendem ausgebildet ist:
Extraktion eines Anatomiemodells (5) aus der wenigstens einen anatomischen Struktur (3),
Extraktion (6) nicht-anatomischer Strukturen (4) aus einer ersten Teilmenge (7) der 2D-Aktualisierungsbilder (2);
Extraktion (13) anatomischer Strukturen (3) aus einer zweiten Teilmenge (8) der 2D-Aktualisierungsbilder (2);
Berechnen eines nicht-anatomischen 3D-Bildes (10) aus wenigstens zwei Teil-Rekonstruktionen (9) aus der ersten Teilmenge (7), wobei die wenigstens zwei Teil-Rekonstruktionen (9) aus der Extraktion (6) der nicht-anatomischen Strukturen (4) berechnet werden und Artefakte der Teil-Rekonstruktionen mittels maschineller Lernverfahren entfernt werden;
Rekonstruktion eines anatomischen 3D-Bildes (11) aus der Extraktion (13) der anatomischen Strukturen (3) aus der zweiten Teilmenge (8);
Registrierung des anatomischen 3D-Bildes (11) mit dem ersten 3D-Bild (1) zur Bestimmung einer Koordinatentransformation (14); und
Erstellung eines Navigationsvolumens (12) aus dem wenigstens einem Anatomiemodell (5) und dem nicht-anatomischen 3D-Bild (10) unter Verwendung der bestimmten Koordinatentransformation (14);
• eine Darstellungseinheit (48), auf welcher die Darstellung des Navigationsvolumens (12) erfolgt.

## Claims

1. A method for operating a medical imaging device for the positionally accurate display of non-anatomical structures during an imaging examination, comprising the following steps:
a.) provisioning of a first 3D image (1) containing at least one anatomical structure (3);
b.) extracting at least one anatomical model (5) from the at least one anatomical structure (3) of the first 3D image (1);
c.) providing at least two 2D update images (2) using the medical imaging device, wherein at least two 2D update images (2) were captured at different times;
d.) extracting (6) non-anatomical structures (4) from a first subset (7) of the 2D update images (2);
e.) extracting (13) anatomical structures (3) from a second subset (8) of the 2D update images (2);
f.) calculating a non-anatomical 3D image (10) from at least two partial reconstructions (9) from the first subset (7), wherein the at least two partial reconstructions (9) are calculated from the extraction (6) of the non-anatomical structures (4) and artifacts of the partial reconstructions are removed by means of machine learning methods;
g.) reconstructing an anatomical 3D image (11) from the extraction (13) of the anatomical structures (3) from the second subset (8);
h.) registering the anatomical 3D image (11) with the first 3D image (1) to determine a coordinate transformation (14);
i.) creating of a navigation volume (12) from the at least one anatomical model (5) and the non-anatomical 3D image (10) using the determined coordinate transformation (14).

2. The method according to claim 1, **characterized in that** the navigation volume (12) is additionally created from the anatomical 3D image (11) using the determined coordinate transformation (14).

3. The method according to claim 1, **characterized in that** the first 3D image (1) is captured with an X-ray device, in particular an X-ray C-arm or a computer tomograph, or with a magnetic resonance tomograph.

4. The method according to one of the preceding claims, **characterized in that** the 2D update images (2) are recorded using an X-ray device, in particular an X-ray C-arm, or a computer tomograph.

5. The method according to one of the preceding claims, **characterized in that**, in the absence of non-anatomical structures (4) in a 2D update image (2) of the first subset (7), the non-anatomical 3D image (10) continues to be reconstructed.

6. The method according to one of the preceding claims, **characterized in that** newly captured 2D update images (21) are added to the first subset (7) and/or second subset (8) and the non-anatomical 3D images (10) and/or the anatomical 3D images (11) are reconstructed in particular at a temporal rate, wherein the temporal rate of reconstruction of the non-anatomical 3D images (10) and/or the anatomical 3D images (11) corresponds to a function of the acquisition rate of the 2D update images (21).

7. The method according to one of the preceding claims, **characterized in that** when a new 2D update image (21) is captured, it is added to the second subset (8) of the 2D update images provided (2) and the anatomical 3D image (11) is at least partially reconstructed anew, whereby temporally older 2D update images are reused or removed for the new reconstruction.

8. The method according to one of the preceding claims, **characterized in that** the method according to the invention, in particular the calculation of the non-anatomical 3D image (10) from the partial reconstructions (9), is performed using a machine learning method, in particular a neural network.

9. The method according to one of the preceding claims, **characterized in that** movements of the patient are taken into account.

10. The method according to one of the preceding claims, **characterized in that** the method, in particular the extraction (6, 13) of the anatomical (3) and/or non-anatomical (4) structures from the 2D update images (2), is performed using at least one machine learning method, in particular a neural network.

11. The method according to one of the preceding claims, **characterized in that** the calculation of the non-anatomical 3D image (10) from the 2D update images (2) is performed by a single machine learning method, in particular a neural network.

12. The method according to one of the preceding claims, **characterized in that** only those non-anatomical structures (4) are extracted which belong to a specific object class or a combination of specific object classes.

13. The method according to one of the preceding claims, **characterized in that** non-anatomical structures (4) belonging to a specific object class or a combination of specific object classes are reconstructed separately.

14. The method according to one of the preceding claims, **characterized in that** the non-anatomical structures (4) are a guide wire and/or catheter.

15. A medical imaging device for performing an imaging examination and displaying non-anatomical structures, comprising:
∘ a provision unit for providing a first 3D image (1), wherein the first 3D image contains at least one anatomical structure;
∘ at least two image chains (41, 42), wherein the image chains are designed to capture 2D update images (2);
∘ a computing unit (47),
∘ wherein the computing unit (47) is adapted to:
extracting an anatomical model (5) from the at least one anatomical structure (3),
extracting (6) non-anatomical structures (4) from a first subset (7) of the 2D update images (2);
extracting (13) anatomical structures (3) from a second subset (8) of the 2D update images (2);
calculating a non-anatomical 3D image (10) from at least two partial reconstructions (9) from the first subset (7), wherein the at least two partial reconstructions (9) are calculated from the extraction (6) of the non-anatomical structures (4) and artifacts of the partial reconstructions are removed by means of machine learning methods;
reconstructing an anatomical 3D image (11) from the extraction (13) of the anatomical structures (3) from the second subset (8);
registering the anatomical 3D image (11) with the first 3D image (1) to determine a coordinate transformation (14); and
creation of a navigation volume (12) from the at least one anatomical model (5) and the non-anatomical 3D image (10) using the determined coordinate transformation (14);
∘ a display unit (48) on which the navigation volume (12) is displayed.

## Revendications

1. Procédé pour faire fonctionner un dispositif d'imagerie médicale pour l'affichage précis en termes de positions de structures non anatomiques pendant un examen d'imagerie, comprenant les étapes suivantes :
a.) la fourniture d'une première image 3D (1) contenant au moins une structure anatomique (3) ;
b.) l'extraction d'au moins un modèle anatomique (5) à partir de l'au moins une structure anatomique (3) de la première image 3D (1) ;
c.) la fourniture d'au moins deux images de mise à jour 2D (2) en utilisant le dispositif d'imagerie médicale, dans lequel les au moins deux images de mise à jour 2D (2) ont été capturées à des temps différents ;
d.) l'extraction (6) de structures non anatomiques (4) à partir d'un premier sous-ensemble (7) des images de mise à jour 2D (2) ;
e.) l'extraction (13) de structures anatomiques (3) à partir d'un second sous-ensemble (8) des images de mise à jour 2D (2) ;
f.) le calcul d'une image 3D non anatomique (10) à partir d'au moins deux reconstructions partielles (9) à partir du premier sous-ensemble (7), dans lequel les au moins deux reconstructions partielles (9) sont calculées à partir de l'extraction (6) des structures non anatomiques (4), et des artefacts des reconstructions partielles sont enlevés au moyen de procédés d'apprentissage automatique ;
g.) la reconstruction d'une image 3D anatomique (11) à partir de l'extraction (13) des structures anatomiques (3) à partir du second sous-ensemble (8) ;
h.) l'enregistrement de l'image 3D anatomique (11) avec la première image 3D (1) pour déterminer une transformation de coordonnées (14) ; et
i.) la création d'un volume de navigation (12) à partir de l'au moins un modèle anatomique (5) et de l'image 3D non anatomique (10) en utilisant la transformation de coordonnées déterminée (14).

2. Procédé selon la revendication 1, **caractérisé en ce que** le volume de navigation (12) est créé de façon additionnelle à partir de l'image 3D anatomique (11) en utilisant la transformation de coordonnées déterminée (14).

3. Procédé selon la revendication 1, **caractérisé en ce que** la première image 3D (1) est capturée à l'aide d'un dispositif à rayons X, en particulier un dispositif à bras C à rayons X ou un tomographe assisté par ordinateur ou un tomographe à résonance magnétique.

4. Procédé selon l'une quelconque des revendications précédentes, **caractérisé en ce que** les images de mise à jour 2D (2) sont enregistrées en utilisant un dispositif à rayons X, en particulier un dispositif à bras C à rayons X ou un tomographe assisté par ordinateur.

5. Procédé selon l'une quelconque des revendications précédentes, **caractérisé en ce que**, en l'absence de structures non anatomiques (4) dans une image de mise à jour 2D (2) du premier sous-ensemble (7), l'image 3D non anatomique (10) continue à être reconstruite.

6. Procédé selon l'une quelconque des revendications précédentes, **caractérisé en ce que** des images de mise à jour 2D nouvellement capturées (21) sont ajoutées au premier sous-ensemble (7) et/ou au second sous-ensemble (8) et les images 3D non anatomiques (10) et/ou les images 3D anatomique (11) sont reconstruites en particulier à une vitesse temporelle, dans lequel la vitesse temporelle de reconstruction des images 3D non anatomiques (10) et/ou des images 3D anatomiques (11) correspond à une fonction de la vitesse d'acquisition des images de mise à jour 2D (21).

7. Procédé selon l'une quelconque des revendications précédentes, **caractérisé en ce que**, lorsqu'une nouvelle image de mise à jour 2D (21) est capturée, elle est ajoutée au second sous-ensemble (8) des images de mise à jour 2D fournies (2) et l'image 3D anatomique (11) est au moins partiellement reconstruite à nouveau, d'où il résulte que des images de mise à jour 2D temporellement plus anciennes sont réutilisées ou enlevées pour la nouvelle reconstruction.

8. Procédé selon l'une quelconque des revendications précédentes, **caractérisé en ce que** le procédé selon l'invention, en particulier le calcul de l'image 3D non anatomique (10) à partir des reconstructions partielles (9), est effectué en utilisant un procédé d'apprentissage automatique, en particulier un réseau neuronal.

9. Procédé selon l'une quelconque des revendications précédentes, **caractérisé en ce que** les mouvements du patient sont pris en compte.

10. Procédé selon l'une quelconque des revendications précédentes, **caractérisé en ce que** le procédé, en particulier l'extraction (6, 13) des structures anatomiques (3) et/ou non anatomiques (4) à partir des images de mise à jour 2D (2), est effectué en utilisant au moins un procédé d'apprentissage automatique, en particulier un réseau neuronal.

11. Procédé selon l'une quelconque des revendications précédentes, **caractérisé en ce que** le calcul de l'image 3D non anatomique (10) à partir des images de mise à jour 2D (2) est effectué au moyen d'un unique procédé d'apprentissage automatique, en particulier un réseau neuronal.

12. Procédé selon l'une quelconque des revendications précédentes, **caractérisé en ce que** seulement les structures non anatomiques (4) qui appartiennent à une classe d'objets spécifique ou à une combinaison de classes d'objets spécifiques sont extraites.

13. Procédé selon l'une quelconque des revendications précédentes, **caractérisé en ce que** les structures non anatomiques (4) qui appartiennent à une classe d'objets spécifique ou à une combinaison de classes d'objets spécifiques sont reconstruites séparément.

14. Procédé selon l'une quelconque des revendications précédentes, **caractérisé en ce que** les structures non anatomiques (4) sont un fil de guidage et/ou un cathéter.

15. Dispositif d'imagerie médicale pour effectuer un examen d'imagerie et pour afficher des structures non anatomiques, comprenant :
∘ une unité de fourniture pour fournir une première image 3D (1), dans lequel la première image 3D contient au moins une structure anatomique ;
∘ au moins deux chaînes d'images (41, 42), dans lequel les chaînes d'images sont conçues pour capturer des images de mise à jour 2D (2) ; et
∘ une unité de calcul informatique (47),
∘ dans lequel l'unité de calcul informatique (47) est adaptée pour :
extraire un modèle anatomique (5) à partir de l'au moins une structure anatomique (3) ;
extraire (6) des structures non anatomiques (4) à partir d'un premier sous-ensemble (7) des images de mise à jour 2D (2) ;
extraire (13) des structures anatomiques (3) à partir d'un second sous-ensemble (8) des images de mise à jour 2D (2) ;
calculer une image 3D non anatomique (10) à partir d'au moins deux reconstructions partielles (9) à partir du premier sous-ensemble (7), dans lequel les au moins deux reconstructions partielles (9) sont calculées à partir de l'extraction (6) des structures non anatomiques (4), et des artefacts des reconstructions partielles sont enlevés au moyen de procédés d'apprentissage automatique ;
reconstruire une image 3D anatomique (11) à partir de l'extraction (13) des structures anatomiques (3) à partir du second sous-ensemble (8) ;
enregistrer l'image 3D anatomique (11) avec la première image 3D (1) pour déterminer une transformation de coordonnées (14) ; et
créer un volume de navigation (12) à partir de l'au moins un modèle anatomique (5) et de l'image 3D non anatomique (10) en utilisant la transformation de coordonnées déterminée (14) ; et
∘ une unité d'affichage (48) sur laquelle le volume de navigation (12) est affiché.
